# EUROPEAN PATENT APPLICATION

(11) **EP 2 385 131 A1**
(43) Date of publication of application: **09.11.2011**
(21) Application number: 10162184.5
(22) Date of filing: 06.05.2010
(51) Int. Cl.: C12P 21/00

(54) **The use of a genetically modified cell line expressing functional asialoglycoprotein receptor in the production of highly sialylated glycoproteins**

(71) Applicant: Charité - Universitätsmedizin Berlin, 10117 Berlin (DE)
(72) Inventor: Berger, Markus, 12209 Berlin (DE); Kaup, Matthias, 13437 Berlin (DE); Lusch, Astrid, 10589 Berlin (DE)
(74) Representative: Ziebig, Marlene

(57) **Abstract**

The present invention is directed to the use of a cell line in the production of highly sialylated glycoprotein, wherein said cell line expresses functional ASGPR protein as well as to a method for the production of highly sialylated glycoproteins, characterized in that such a cell line is used.

## Description

More and more proteins are used as active ingredients in pharmaceuticals (so called biopharmaceuticals or biologics) for treatment of various diseases. In order to allow for controlled production, these proteins are usually produced by genetically modified cell lines, which have been modified to express and preferably secrete the desired proteins, so called production cell lines. Usually, production cell lines for the manufacture of proteins for medical use are of mammalian origin, e.g. hamster, mouse, rat, dog, duck or human, preferably of rodent or human origin.

In order to provide fully functional proteins, it is important that produced proteins exhibit the correct conformation and show adequate posttranslational modifications. In particular posttranslational modifications can be crucial for stability, solubility, plasma half-life, antigenicity and biological activity of the proteins produced. Glycosylation has been identified as a posttranslational modification which has a major impact on these characteristics. Thus, there have already been attempts to develop cell lines which have been optimized for glycosylation of proteins produced by cells of these cell lines.

One important characteristic influencing the plasma half life or stability of a protein in the blood stream is the degree of sialylation of the glycosylated protein (glycoprotein). Sialic acids are negatively charged sugar molecules and represent the terminal residues of complex glycan structures of glycoproteins. The sugar molecules of the glycan structure residing "under" or being capped by these terminal sialic acid residues are masked or protected from detrimental effects of the blood serum. Glycoproteins that show no or only incomplete sialylation of its glycan structures exhibit a reduced plasma half-life or stability in blood. One important mechanism leading to rapid blood clearance of glycoproteins with such incomplete sialylation is based on the presence of the asialoglycoprotein receptor, ASGPR.

ASGPR is predominantly expressed on cells in the liver and can bind complex glycan structures of glycoproteins wherein the terminal sialic acid residue of the glycan structure is removed, so called asialoglycoproteins. Upon binding, the bound asialoglycoproteins are internalized into the cells and degraded. Thus, glycoproteins with incomplete sialylation are efficiently extracted from the blood and are no longer available to exert the desired biological or medical function, whereas glycoproteins with a higher degree of sialylation or that carry solely fully sialylated glycan structures are less prone to binding to ASGPR and therefore show prolonged plasma half life than less sialylated glycoproteins.

In the past, it has been tried to develop cell lines that allow for the production of glycoproteins with a high degree of sialylation. Most attempts were directed to modify enzymes and proteins of a cell line that are directly involved in sialylation of the glycoproteins to be expressed. Thus, up to now the focus was on influencing processes, enzymes and proteins that form part of the biosynthesis of glycan structures and/or glycoproteins. Examples of such means and methods are described in US 5,047,335, WO 2005/080585, EP 1 900 750, EP 1 911 766 and US 2009/0298120.

Although these attempts can yield to a higher degree of sialylation of the produced glycoproteins, the product of these cells still contains a significant fraction of the desired glycoprotein that exhibit no or only a limited degree of sialylation. In order to further improve production methods for glycoproteins, there remains a need for additional, novel techniques.

Thus, it is an object of the present invention to overcome or alleviate at least one of the disadvantages of the prior art. In particular it is an object of the present invention to provide new methods and means for efficient production of highly sialylated glycoproteins.

This object is achieved by the use of a cell line in the production of highly sialylated glycoprotein, wherein said cell line expresses functional ASGPR protein.

Preferably, the cell line of the invention is used in the production of highly sialylated glycoprotein, wherein the highly sialylated glycoprotein to be produced is not a functional ASGPR protein or a part thereof.

Preferably a cell line is used, wherein the functional ASGPR protein comprises or consists of:
- an amino acid sequence being at least 80% identical to the amino acid sequence of human ASGPR-I with SEQ ID No. 1; and
- an amino acid sequence being at least 80% identical to an amino acid sequence of human ASGPR-11 with SEQ ID No. 2, SEQ ID No. 5 or SEQ ID No. 6; wherein sequence identity is determined over a sequence length of at least 100 consecutive amino acids of SEQ ID No. 1, 2, 5 or 6 respectively.

Said cell line can be a genetically modified cell line, wherein the cell line is genetically modified to express functional ASGPR protein.

The inventors have surprisingly found that the use of a cell line that expresses functional ASGPR protein in the production of glycoproteins leads to efficient enrichment of highly sialylated glycoproteins already during the process of glycoprotein production, thereby obviating the need for laborious post-production treatment.

For the purpose of the present invention, the use in the production of highly sialylated glycoprotein refers to any use, wherein the cell line of the invention is not primarily or solely employed to monitor or test the quality of a product containing glycoproteins, but is employed in such a way, that the overall content of highly sialylated glycoprotein is directly and actively improved in the product obtained. The terms "directly" and "actively" are used to denote that the improvement in content of highly sialylated glycoprotein is a direct result of the use of a cell line of the invention during production of glycoprotein and does not exhaust simply in testing the quality of already produced glycoprotein. Furthermore, said improvement in content of highly sialylated glycoprotein is at least in part a direct result of cellular activity of a cell line of the invention that comprises binding of unsialylated or incompletely sialylated glycoprotein to the functional ASGPR protein expressed by the cell line of the invention.

Thus, in a preferred embodiment the use of the present invention comprises a use, wherein the cell line is employed to directly and actively improve the production of highly sialylated glycoprotein.

According to the present invention a cell line is used. The term "cell line" is used herein to denote immortalized cells of a common cell type and origin that can proliferate essentially indefinitely. Preferably the term "cell line" refers to cells that are morphologically and genetically essentially uniform and substantially genetically stable. The cell line of the invention is preferably a cell line that has been used or described previously for production of biologics (biopharmaceuticals like e.g. peptides, proteins, glycoproteins, enzymes, hormones, vaccines etc.) or is derived therefrom. The cell line of the invention is preferably of mammalian origin, more preferably of hamster, mouse, rat, dog, duck or human origin, most preferably of rodent or human origin. In particular, the cell line of the invention can be derived from HEK293, CHO, BHK, Vero, NSO and/or a cell or cell line derived therefrom. The corresponding ATCC numbers are: HEK293: CRL-1573, CHO-K1: CCL-61, BHK-21[C-13]: CCL-1 0, Vero: CCL-81, NSO-GT12: CCL-12066.

The cell line of the invention can be a genetically modified cell line, wherein the cell line has been genetically modified to express functional ASGPR protein.

As used herein the term "genetically modified cell line" refers to a cell line that has been modified by alteration of its genetic configuration or nucleic acid content. Said modification may e.g. comprise the introduction of nucleic acid molecules or the alteration or mutation of the genetic configuration or genome of the cells or of the cell line. Said modification may be performed in such a way that the resulting cell line is either transiently or stably genetically modified.

The genetically modified cell line of the invention is preferably derived from a cell line that prior to genetic modification does not exhibit detectable expression of functional ASGPR protein. However, the genetically modified cell line of the invention may also be derived from a cell line that already expresses endogenous functional ASGPR protein.

The cell line of the present invention can be genetically modified to express functional ASGPR protein. Said modification can be achieved by stably or transiently transfecting cells of a cell line with one or more nucleic acid molecules comprising nucleic acid sequences encoding for a functional ASGPR protein. The skilled person is well aware of the degeneracy of the genetic code and of the fact that more than one nucleic acid sequence can encode for one and the same amino acid sequence. When designing a nucleic acid sequence that encodes for at least one subunit of a functional ASGPR protein, the skilled person can take into account the preferred codon usage of the origin where the cell line to be genetically modified is derived from. The nucleic sequences encoding for a functional ASGPR protein may be present within one single nucleic acid molecule or the nucleic acid sequences encoding for the subunits of functional ASGPR protein may be comprised on one or more separate nucleic acid molecules.

In particular, said one or more nucleic acid molecules comprise or consist of:
- a nucleic acid sequence being at least 80% identical to the nucleic acid sequences of human ASGPR-I with SEQ ID No. 3, preferably at least 90%, more preferably at least 95%, most preferably 99% identical to SEQ ID No. 3; and
- a nucleic acid sequence being at least 80% identical to the nucleic acid sequences of human ASGPR-II with SEQ ID No. 4, 7 or 8, preferably at least 90%, more preferably at least 95%, most preferably 99% identical to SEQ ID No. 4, 7 or 8,
wherein sequence identity is determined over a sequence length of at least 100 consecutive nucleic acids of SEQ ID No. 3, 4, 7 or 8 respectively, preferably sequence identity is determined over the whole sequence length of SEQ ID No. 3, 4, 7 or 8 respectively.

Sequence identity is expressed as % identity over a given sequence length. Sequence identity can be calculated using the blast algorithm available under http://blast.ncbi.nlm.nih.gov/Blast.cgi. Sequence identity for nucleotide sequences is calculated with the BlastN algorithm, whereas sequence identity of amino acid sequences is calculated with the BlastP algorithm.

Preferably, said one or more nucleic acid molecules comprise or consist of the nucleic acid sequences of human ASGPR-I with SEQ ID No. 3 and of one of human ASGPR-II with SEQ ID No. 4, 7 or 8.

Said one or more nucleic acid molecules can comprise further nucleic acid sequences that may enable or support expression of functional ASGPR protein. Said further nucleic acid sequences may comprise promoter sequences and/or other regulatory nucleic acid sequences that can impact transcription and/or translation of functional ASGPR protein or parts thereof.

The genetically modified cell line of the invention is genetically modified to express functional ASGPR protein. The ASGPR protein is a receptor of the lectin family that is able to bind glycoproteins, wherein the terminal sialic acid residue of a glycan structure is missing or removed. Functional ASGPR protein is composed of different protein subunits. Functional human ASGPR protein is composed of two different protein subunits I and II, whereas functional ASGPR protein from other species may comprise more than two subunits, e.g. functional ASGPR protein from mouse and rat comprises three different protein subunits.

There are three isoforms of human ASGPR-II subunit which exhibit an amino acid sequence depicted in SEQ ID No. 2, SEQ ID No. 5 and SEQ ID No. 6, respectively. The nucleic acid sequence corresponding to human ASGPR-II subunit with the amino acid sequence of SEQ ID No. 2 is provided as SEQ ID No. 4. The nucleic acid sequence corresponding to human ASGPR-II subunit with the amino acid sequence of SEQ ID No. 5 is provided as SEQ ID No. 7. The nucleic acid sequence corresponding to human ASGPR-II subunit with the amino acid sequence of SEQ ID No. 6 is provided as SEQ ID No. 8.

A cell or cell line expresses a functional ASGPR protein in the sense of the present invention if said cell or cell line expresses or is genetically modified to express all relevant ASGPR subunits (e.g. subunits I and II for human ASGPR protein) in such a way that the resulting cell or cell line displays ASGPR protein on the cell surface and shows a detectable rate of binding and internalisation of asialoglycoproteins. In a preferred embodiment, the cell or cell line genetically modified to express functional ASGPR protein exhibits an internalisation rate for asialoglycoproteins, like e.g. alpha-1-antitrypsin (A1AT) or alpha-1-acid glycoprotein, that is increased by at least a factor of 2 compared to the parent cell or cell line which lacks said genetic modification. In a particularly preferred embodiment the internalisation rate is increased by at least a factor of 5, more preferably by at least a factor of 10.

For the purpose of the present invention, the internalisation rate can be determined by:
(1) contacting cells with a predetermined amount of an unsialylated glycoprotein, incompletely sialylated glycoprotein and/or of a mixture comprising unsialylated or incompletely sialylated glycoprotein, preferably the unsialylated or incompletely sialylated glycoprotein is alpha-1-antitrypsin (A1AT) or alpha1-acid glycoprotein,
(2) incubating the cells with the unsialylated or incompletely sialylated glycoprotein and
(3) determining the amount of non-internalised unsialylated and/or incompletely sialylated glycoprotein. For that purpose the unsialylated glycoprotein may be labeled with a label that allows for easy detection and quantification, e.g. by a radioactive label or a fluorescence label.

The internalisation rate can be expressed as:
(predetermined amount of unsialylated and/or incompletely sialylated glycoprotein minus determined amount of non-internalised unsialylated and/or incompletely sialylated glycoprotein) / time unit.

Preferably the cell line of the invention expresses functional ASGPR protein, wherein the functional ASGPR protein comprises or consists of:
- an amino acid sequence being at least 80% identical to the amino acid sequence of human ASGPR-I with SEQ ID No. 1, preferably at least 90%, more preferably at least 95%, most preferably 99% identical to SEQ ID No. 1; and
- an amino acid sequence being at least 80% identical to an amino acid sequence of human ASGPR-II with SEQ ID No. 2, 5 or 6, preferably at least 90%, more preferably at least 95%, most preferably 99% identical to SEQ ID No. 2, 5 or 6,
wherein sequence identity is determined over a sequence length of at least 100 consecutive amino acids of SEQ ID No. 1, 2, 5 or 6 respectively, preferably sequence identity is determined over the whole sequence length of SEQ ID No. 1, 2, 5 or 6 respectively.

Preferably, the cell line of the invention expresses functional ASGPR protein, wherein the functional ASGPR protein comprises or consists of:
- the amino acid sequence of human ASGPR-I with SEQ ID No. 1; and
- an amino acid sequence of human ASGPR-II with SEQ ID No. 2, 5 or 6.

The cell line of the present invention can be used to eliminate unsialylated or incompletely sialylated glycoproteins expressed and secreted from a separate production cell line, wherein the production cell line is different from the cell line of the present invention. In this case the cell line of the invention is used as a "scavenger" in order to eliminate unwanted by-products (i.e. unsialylated or incompletely sialylated versions of the glycoprotein to be produced) from the primary product of the producer cell line. In order to achieve said elimination it is possible to co-culture the production cell line and the cell line of the invention within the same culture cavity, so that secretion of primary glycoprotein products from the producer cell line occurs simultaneously with elimination of incompletely sialylated glycoproteins from said primary product resulting in a secondary product being enriched for fully sialylated glycoprotein to be produced.

Alternatively cells of the cell line of the invention may be contacted with the primary product of the producer cell line in order to eliminate unsialylated or incompletely sialylated versions of the glycoprotein to be produced. This can e.g. be done by contacting cells of the cell line of the invention with supernatant and/or conditioned media of the producer cell line containing the glycoprotein to be produced or with any other probe, e.g. liquid probe, containing the primary products of the producer cell line. This alternative approach allows culturing of the producer cell line and the cell line of the invention under different culture conditions. Preferably both cell lines can be cultured under the respective optimal conditions. Thus, the producer cell line can be cultured under conditions optimized for expression and secretion of the glycoprotein to be produced, whereas the cell line of the invention can be cultured under conditions optimized for binding and internalisation of unsialylated or incompletely sialylated glycoproteins.

Instead of using two separate cell lines for production of the glycoprotein and enrichment of highly sialylated glycoprotein, both functions may be combined within one single cell line. Therefore, the cell line of the invention can be (further) genetically modified to express and secrete the glycoprotein to be produced. The use of only one single cell line reduces the need for optimization of culture conditions, since conditions for only one cell line have to be determined. Glycoprotein production and enrichment for highly sialylated glycoprotein can be done simultaneously in one step and one culture cavity. The whole cell mass in the culture vessel is available for production of the desired glycoprotein. In this case, the cell line of the invention can recycle the internalized unsialylated or incompletely sialylated glycoprotein and can use its components for the production of fully sialylated or highly sialylated glycoprotein. Thus, the resources available will be exploited more economically.

In a preferred embodiment the cell line of the invention and, optionally, the production cell line are compatible with culture in suspension, preferably with culture in a suspension bioreactor. Alternatively, the cell line of the invention, the producer cell line or both can be cultured under adherent culture conditions.

The present invention is also directed to a method for the production of highly sialylated glycoproteins, characterized in that a cell line of the invention is used.

In a preferred method of the invention, a cell line is used, wherein said cell line expresses functional ASGPR protein, wherein the functional ASGPR protein preferably comprises or consists of:
- an amino acid sequence being at least 80% identical to the amino acid sequence of human ASGPR-I with SEQ ID No. 1; and
- an amino acid sequence being at least 80% identical to an amino acid sequence of human ASGPR-II with SEQ ID No. 2, 5 or 6;
wherein sequence identity is determined over a sequence length of at least 100 consecutive amino acids of SEQ ID No. 1, 2, 5 or 6 respectively.

The cell lines of the invention defined in detail above and in particular the genetically modified cell lines of the invention can be used in the method for the production of highly sialylated glycoproteins of the invention.

In the method of the invention, the cell line can be (further) genetically modified to express and secrete the glycoprotein to be produced.

Alternatively or in addition, in the method of the invention the cell line may be used in combination with a production cell line, which expresses and secretes the glycoprotein to be produced.

Preferably the method of the invention comprises the step of contacting a probe containing the glycoprotein to be produced with the cell line of the invention.

In a preferred embodiment, the method of the present invention comprises the steps:
(i) providing means to express, glycosylate and secrete the glycoprotein to be produced, preferably such means may be seen in a producer cell line and/or in a cell line of the invention further modified to express and secrete the desired glycoprotein;
(ii) contacting the secreted glycoprotein with the cell line of the invention, so that unsialylated glycoprotein can be absorbed by cells of the cell line of the invention; and
(iii) isolating the secreted glycoprotein that is not absorbed by cells of the cell line of the invention.

Preferably at least the contacting of the secreted glycoprotein with the cell line of the invention is performed under suspension culture conditions, preferably in a bioreactor.

Below the invention is described in further detail by way of examples.

### FIGURES:

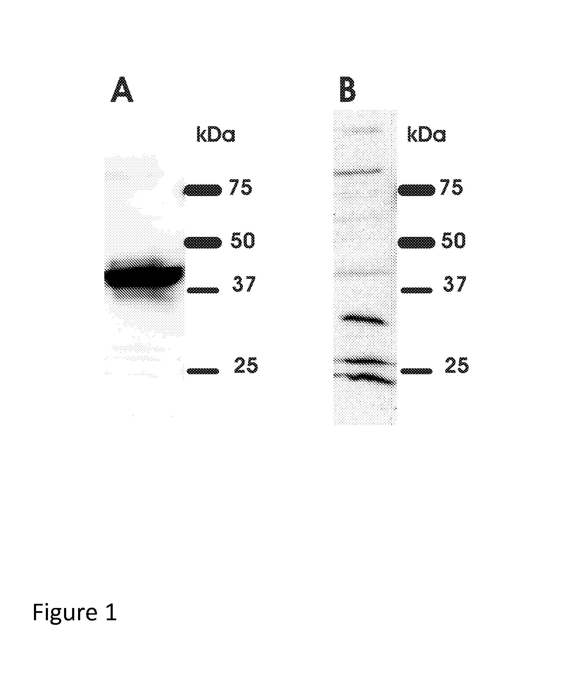

- Figure 1 shows: a western blot analysis of expressed ASGPR subunits I and II in HEK cells stably transfected to express functional ASGPR protein. A: Antibody detects ASGPR-I with a molecular mass of 43 kDa. B: The antibody for the ASGPR-II subunit shows a specific band of 32kDa (both antibodies: Aviva Systems Biology, San Diego, CA, USA)

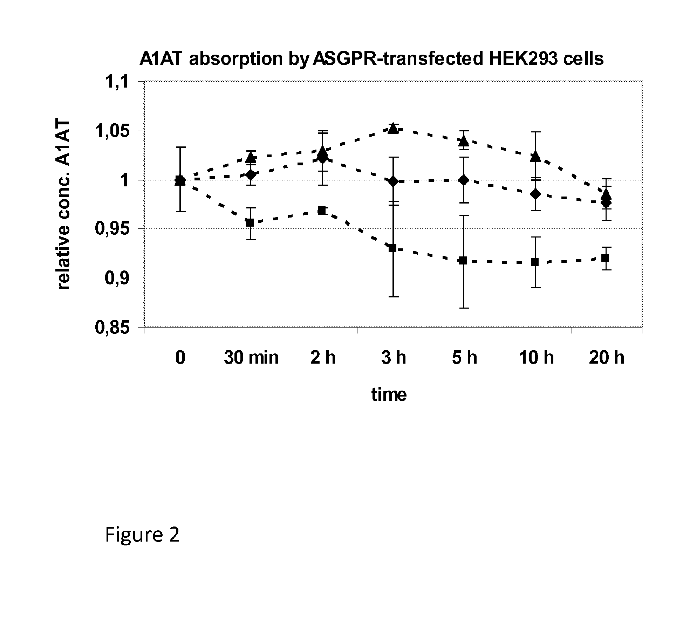

- Figure 2 shows: Relative absorption of A1AT wildtype protein (protein mixture comprising unsialylated, incompletely sialylated and fully sialylated A1AT), out of the media in the presence of different cells. Triangles: A1AT in the presence of HEK293 wildtype cells. Rhombuses: A1AT in the presence of HEK293 cells with the ASGPR-I subunit. Squares: Inreased absorption due to the uptake of A1AT by HEK293 presenting the functional ASGPR (ASGPR I and II). The different cell types were grown to confluence and seeded with (∼))1x10E5 cells/well and were incubated with a given concentration of recombinant A1AT wildtype protein. After different time steps the concentration of A1AT in the media was analysed by ELISA.

### EXAMPLES:

### 1.1 Cloning of A1AT and ASGPR-I and-II

The human A1AT-cDNA was cloned into pcDNA3.1 zeo (+) vector from Invitrogen (Darmstadt, Germany). ASGPR-I and ASGPR-II isoform 2 cDNA was obtained from ImaGenes (Berlin, Germany) and cloned into pcDNA3.1 zeo (+) or pcDNA3.1 hygro (+) from Invitrogen.

### 1.2 Transfection of HEK293 cells and selection

HEK293 cells were cultured at 5% CO₂ atmosphere and 37 °C in DMEM with Glutamin supplemented with penicillin / streptomycin and 5% FCS. Transfections were performed with Lipofectamine according to the manufacture's instructions and cells selected in the presence of zeocin (A1AT or ASGPR-I) or hygromycin (ASGPR-II).

### 1.3 Expression and purification of AIATwt

Stably transfected HEK293 cells were grown to 50% confluency, washed two times with PBS and cultivated in AEM medium for 24h. Subsequently the medium was removed and the cells resuspended in fresh AEM medium and grown in shaking flasks for ten days. The cell cultures were centrifuged, the supernatant filtrated by a 0.22 µm filter and diluted 1:2 in water. The A1AT protein were purified then by anion exchange chromatography (MonoQ 5/50 GL, buffer A 0.5 x PBS, buffer B 0.5 x PBS + 1 M NaCl) following by size exclusion chromatography (Superdex 200 10/300 GL, running buffer 0.5 x PBS).

### 1.4 SDS -Polyacrylamide gel electrophoresis, electroblotting and immunostaining

Samples were prepared according to Laemmli (Laemmli, U.K. (1970) Nature 227, 680-5) and proteins were transferred to a nitrocellulose membrane. For this, electrotransfer of proteins was performed in a tank trans-blot cell (Bio-Rad, Richmond, CA, USA) with blotting buffer (25mM Tris, 114 mM glycine, 10 % (v/v) ethanol). After blotting, the membrane was blocked with PBS containing 5% dry skim milk. ASGPR-I and ASGPR-II were detected by polyclonal Anti-ASGRP-I and -II antibodies (Aviva Systems Biology, San Diego, CA, USA) respectively, followed by secondary antibody detection (peroxidase-conjugated goat anti rabbit IgG, Jackson Immuno Research Laboratories, West Grove, PA, USA) using the chemiluminescence reaction and the versa doc system (Bio-Rad, Richmond, CA, USA).

### 1.5 Clearance-Assay

Stably ASGPR-I and doubly ASGPR-I and -II transfected HEK293 cells as well as non- transfected HEK293 wild-type cells were seeded at ∼1X10⁴ cells in 96-well plates and grown for two days under standard culture conditions. Then purified recombinant A1AT was added to a final concentration of 6 µg/ml to each well. At indicated times 100 µl of the supernatants were removed (at every time from different wells but equally treated) and A1AT content analyzed by A1AT-ELISA. Data was calculated as percent of the starting A1AT concentration and interpreted as absorbance of A1AT by the HEK293 cells.

### 1.6 AIAT-ELISA

Using a 96-well micro plate, each well was coated with 100 µl of polyclonal rabbit anti-human A1AT antibody (0.14 µg/ml in PBS) for 16 h at 4 °C. All following incubation steps were carried out at room temperature. The wells were subsequently blocked with 200 µl of 1 % (w/v) BSA and 0.2 % (v/v) Tween 20 in PBS for 2 h. 100 µl of standard and samples were pipetted in triplicate and incubated for 2 h. The plate was washed with PBS containing 0.2 % (v/v) Tween 20 and bound A1AT detected with 100 µl monoclonal sheep anti-human A1AT HRP conjugated antibody.

Results are shown in Figures 1 and 2.

In Figure 1, it is demonstrated that the HEK293 cells stably transfected to express functional ASGPR protein indeed exhibit expression of ASGPR-I and -II subunits.

In Figure 2, it is shown that HEK293 cells stably transfected to express functional ASGPR protein over time eliminate recombinant A1AT protein from supernatant containing a mixture of unsialylated, incompletely sialylated and highly sialylated A1AT, whereas HEK293 cell that lack said genetic modification do not show such an elimination.

Thus, it can be concluded that HEK293 cells genetically modified to express functional ASGPR protein are capable of quantitatively eliminating unsialylated and/or incompletely sialylated glycoprotein from a supernatant or conditioned medium.

## Claims

1. Use of a cell line in the production of highly sialylated glycoprotein, wherein said cell line expresses functional ASGPR protein.

2. Use according to claim 1, wherein the cell line is employed to directly and actively improve the production of highly sialylated glycoprotein.

3. Use according to one of the preceding claims, wherein the functional ASGPR protein comprises or consists of:
- an amino acid sequence being at least 80% identical to the amino acid sequence of human ASGPR-I with SEQ ID No. 1; and
- an amino acid sequence being at least 80% identical to an amino acid sequence of human ASGPR-II with SEQ ID No. 2, SEQ ID No. 5 or SEQ ID No. 6; wherein sequence identity is determined over a sequence length of at least 100 consecutive amino acids of SEQ ID No. 1, 2, 5 or 6 respectively.

4. Use according to one of the preceding claims, wherein the functional ASGPR protein comprises or consists of:
- the amino acid sequence of human ASGPR-I with SEQ ID No. 1; and
- the amino acid sequence of human ASGPR-II with one of SEQ ID No. 2, SEQ ID No. 5 or SEQ ID No. 6.

5. Use according to one of the preceding claims, wherein the cell line is a genetically modified cell line which is genetically modified to express functional ASGPR protein.

6. Use according to claim 5, wherein the genetically modified cell line is transiently or stably transfected with one or more nucleic acid molecules comprising nucleic acid sequences encoding for a functional ASGPR protein.

7. Use according to claim 5 or 6, wherein the cell line genetically modified to express functional ASGPR protein exhibits an internalisation rate for asialoglycoproteins that is increased at least by a factor of 2 compared to the respective parent cell line which lacks said genetic modification.

8. Use according to one of the preceding claims, wherein the cell line is genetically modified to express and secrete the glycoprotein to be produced.

9. Use according to one of the preceding claims, wherein the cell line is used in combination with a separate production cell line, which expresses and secretes the glycoprotein to be produced.

10. Use according to one of the preceding claims, wherein the cell line and, optionally, the production cell line are used in suspension culture.

11. Method for the production of highly sialylated glycoproteins, **characterized in that** a cell line is used, wherein said cell line expresses functional ASGPR protein.

12. The method of claim 11, wherein the functional ASGPR protein comprises or consists of:
- an amino acid sequence being at least 80% identical to the amino acid sequence of human ASGPR-I with SEQ ID No. 1; and
- an amino acid sequence being at least 80% identical to an amino acid sequence of human ASGPR-II with SEQ ID No. 2, SEQ ID No. 5 or SEQ ID No. 6; wherein sequence identity is determined over a sequence length of at least 100 consecutive amino acids of SEQ ID No. 1, 2, 5 or 6 respectively.

13. The method according to claim 11 or 12, comprising the steps:
(i) providing means to express, glycosylate and secrete the glycoprotein to be produced;
(ii) contacting the secreted glycoprotein with the cell line, so that unsialylated glycoprotein can be absorbed by cells of the cell line; and
(iii) isolating the secreted glycoprotein that is not absorbed by cells of the cell line.

14. The method according to claim 13, wherein at least the contacting of the secreted glycoprotein with the cell line is performed under suspension culture conditions.

15. The method according to one of claims 11 to 14, wherein the cell line is a genetically modified cell line which is genetically modified to express functional ASGPR protein.


SEQUENCE LISTING

<110>  Charite Universitätsmedizin Berlin

<120>  The use of a genetically modified cell line expressing functional
        ASGPR protein in the production of highly sialylated
       glycoproteins

<130>  P07767EP

<160>  8

<170>  PatentIn version 3.3

<210>  1
<211>  291
<212>  PRT
<213>  homo sapiens

<400>  1

Met Thr Lys Glu Tyr Gln Asp Leu Gln His Leu Asp Asn Glu Glu Ser
1               5                   10                  15

Asp His His Gln Leu Arg Lys Gly Pro Pro Pro Pro Gln Pro Leu Leu
            20                  25                  30

Gln Arg Leu Cys Ser Gly Pro Arg Leu Leu Leu Leu Ser Leu Gly Leu
        35                  40                  45

Ser Leu Leu Leu Leu Val Val Val Cys Val Ile Gly Ser Gln Asn Ser
    50                  55                  60

Gln Leu Gln Glu Glu Leu Arg Gly Leu Arg Glu Thr Phe Ser Asn Phe
65                  70                  75                  80

Thr Ala Ser Thr Glu Ala Gln Val Lys Gly Leu Ser Thr Gln Gly Gly
                85                  90                  95

Asn Val Gly Arg Lys Met Lys Ser Leu Glu Ser Gln Leu Glu Lys Gln
            100                 105                 110

Gln Lys Asp Leu Ser Glu Asp His Ser Ser Leu Leu Leu His Val Lys
        115                 120                 125

Gln Phe Val Ser Asp Leu Arg Ser Leu Ser Cys Gln Met Ala Ala Leu
    130                 135                 140

Gln Gly Asn Gly Ser Glu Arg Thr Cys Cys Pro Val Asn Trp Val Glu
145                 150                 155                 160

His Glu Arg Ser Cys Tyr Trp Phe Ser Arg Ser Gly Lys Ala Trp Ala
                165                 170                 175

Asp Ala Asp Asn Tyr Cys Arg Leu Glu Asp Ala His Leu Val Val Val
            180                 185                 190

Thr Ser Trp Glu Glu Gln Lys Phe Val Gln His His Ile Gly Pro Val
        195                 200                 205

Asn Thr Trp Met Gly Leu His Asp Gln Asn Gly Pro Trp Lys Trp Val
    210                 215                 220

Asp Gly Thr Asp Tyr Glu Thr Gly Phe Lys Asn Trp Arg Pro Glu Gln
225                 230                 235                 240

Pro Asp Asp Trp Tyr Gly His Gly Leu Gly Gly Gly Glu Asp Cys Ala
                245                 250                 255

His Phe Thr Asp Asp Gly Arg Trp Asn Asp Asp Val Cys Gln Arg Pro
            260                 265                 270

Tyr Arg Trp Val Cys Glu Thr Glu Leu Asp Lys Ala Ser Gln Glu Pro
        275                 280                 285

Pro Leu Leu
    290

<210>  2
<211>  311
<212>  PRT
<213>  homo sapiens

<400>  2

Met Ala Lys Asp Phe Gln Asp Ile Gln Gln Leu Ser Ser Glu Glu Asn
1               5                   10                  15

Asp His Pro Phe His Gln Gly Glu Gly Pro Gly Thr Arg Arg Leu Asn
            20                  25                  30

Pro Arg Arg Gly Asn Pro Phe Leu Lys Gly Pro Pro Pro Ala Gln Pro
        35                  40                  45

Leu Ala Gln Arg Leu Cys Ser Met Val Cys Phe Ser Leu Leu Ala Leu
    50                  55                  60

Ser Phe Asn Ile Leu Leu Leu Val Val Ile Cys Val Thr Gly Ser Gln
65                  70                  75                  80

Ser Glu Gly His Arg Gly Ala Gln Leu Gln Ala Glu Leu Arg Ser Leu
                85                  90                  95

Lys Glu Ala Phe Ser Asn Phe Ser Ser Ser Thr Leu Thr Glu Val Gln
            100                 105                 110

Ala Ile Ser Thr His Gly Gly Ser Val Gly Asp Lys Ile Thr Ser Leu
        115                 120                 125

Gly Ala Lys Leu Glu Lys Gln Gln Gln Asp Leu Lys Ala Asp His Asp
    130                 135                 140

Ala Leu Leu Phe His Leu Lys His Phe Pro Val Asp Leu Arg Phe Val
145                 150                 155                 160

Ala Cys Gln Met Glu Leu Leu His Ser Asn Gly Ser Gln Arg Thr Cys
                165                 170                 175

Cys Pro Val Asn Trp Val Glu His Gln Gly Ser Cys Tyr Trp Phe Ser
            180                 185                 190

His Ser Gly Lys Ala Trp Ala Glu Ala Glu Lys Tyr Cys Gln Leu Glu
        195                 200                 205

Asn Ala His Leu Val Val Ile Asn Ser Trp Glu Glu Gln Lys Phe Ile
    210                 215                 220

Val Gln His Thr Asn Pro Phe Asn Thr Trp Ile Gly Leu Thr Asp Ser
225                 230                 235                 240

Asp Gly Ser Trp Lys Trp Val Asp Gly Thr Asp Tyr Arg His Asn Tyr
                245                 250                 255

Lys Asn Trp Ala Val Thr Gln Pro Asp Asn Trp His Gly His Glu Leu
            260                 265                 270

Gly Gly Ser Glu Asp Cys Val Glu Val Gln Pro Asp Gly Arg Trp Asn
        275                 280                 285

Asp Asp Phe Cys Leu Gln Val Tyr Arg Trp Val Cys Glu Lys Arg Arg
    290                 295                 300

Asn Ala Thr Gly Glu Val Ala
305                 310

<210>  3
<211>  876
<212>  DNA
<213>  homo sapiens

<400>  3
atgaccaagg agtatcaaga ccttcagcat ctggacaatg aggagagtga ccaccatcag      60

ctcagaaaag ggccacctcc tccccagccc ctcctgcagc gtctctgctc cggacctcgc     120

ctcctcctgc tctccctggg cctcagcctc ctgctgcttg tggttgtctg tgtgatcgga     180

tcccaaaact cccagctgca ggaggagctg cggggcctga gagagacgtt cagcaacttc     240

acagcgagca cggaggccca ggtcaagggc ttgagcaccc agggaggcaa tgtgggaaga     300

aagatgaagt cgctagagtc ccagctggag aaacagcaga aggacctgag tgaagatcac     360

tccagcctgc tgctccacgt gaagcagttc gtgtctgacc tgcggagcct gagctgtcag     420

atggcggcgc tccagggcaa tggctcagaa aggacctgct gcccggtcaa ctgggtggag 480

cacgagcgca gctgctactg gttctctcgc tccgggaagg cctgggctga cgccgacaac 540

tactgccggc tggaggacgc gcacctggtg gtggtcacgt cctgggagga gcagaaattt 600

gtccagcacc acataggccc tgtgaacacc tggatgggcc tccacgacca aaacgggccc 660

tggaagtggg tggacgggac ggactacgag acgggcttca agaactggag gccggagcag 720

ccggacgact ggtacggcca cgggctcgga ggaggcgagg actgtgccca cttcaccgac 780

gacggccgct ggaacgacga cgtctgccag aggccctacc gctgggtctg cgagacagag 840

ctggacaagg ccagccagga gccacctctc ctttaa 876

<210> 4
<211> 936
<212> DNA
<213> homo sapiens

<400> 4
atggccaagg actttcaaga tatccagcag ctgagctcgg aggaaaatga ccatcctttc 60

catcaaggtg aggggccagg cactcgcagg ctgaatccca ggagaggaaa tccatttttg 120

aaagggccac ctcctgccca gcccctggca cagcgtctct gctccatggt ctgcttcagt 180

ctgcttgccc tgagcttcaa catcctgctg ctggtggtca tctgtgtgac tgggtcccaa 240

agtgagggtc acagaggtgc acagctgcaa gccgagctgc ggagcctgaa ggaagctttc 300

agcaacttct cctcgagcac cctgacggag gtccaggcaa tcagcaccca cggaggcagc 360

gtgggtgaca agatcacatc cctaggagcc aagctggaga aacagcagca ggacctgaaa 420

gcagatcacg atgccctgct cttccatctg aagcacttcc ccgtggacct gcgcttcgtg 480

gcctgccaga tggagctcct ccacagcaac ggctcccaaa ggacctgctg ccccgtcaac 540

tgggtggagc accaaggcag ctgctactgg ttctctcact ccgggaaggc ctgggctgag 600

gcggagaagt actgccagct ggagaacgca cacctggtgg tcatcaactc ctgggaggag 660

cagaaattca ttgtacaaca cacgaacccc ttcaatacct ggataggtct cacggacagt 720

gatggctctt ggaaatgggt ggatggcaca gactataggc acaactacaa gaactgggct 780

gtcactcagc cagataattg gcacgggcac gagctgggtg gaagtgaaga ctgtgttgaa 840

gtccagccgg atggccgctg gaacgatgac ttctgcctgc aggtgtaccg ctgggtgtgt 900

gagaaaaggc ggaatgccac cggcgaggtg gcctga 936

<210> 5
<211> 287
<212> PRT
<213> homo sapiens

<400> 5

Met Ala Lys Asp Phe Gln Asp Ile Gln Gln Leu Ser Ser Glu Glu Asn
1               5                   10                  15

Asp His Pro Phe His Gln Gly Pro Pro Pro Ala Gln Pro Leu Ala Gln

20                  25                  30

Arg Leu Cys Ser Met Val Cys Phe Ser Leu Leu Ala Leu Ser Phe Asn
        35                  40                  45

Ile Leu Leu Leu Val Val Ile Cys Val Thr Gly Ser Gln Ser Ala Gln
    50                  55                  60

Leu Gln Ala Glu Leu Arg Ser Leu Lys Glu Ala Phe Ser Asn Phe Ser
65                  70                  75                  80

Ser Ser Thr Leu Thr Glu Val Gln Ala Ile Ser Thr His Gly Gly Ser
                85                  90                  95

Val Gly Asp Lys Ile Thr Ser Leu Gly Ala Lys Leu Glu Lys Gln Gln
            100                 105                 110

Gln Asp Leu Lys Ala Asp His Asp Ala Leu Leu Phe His Leu Lys His
        115                 120                 125

Phe Pro Val Asp Leu Arg Phe Val Ala Cys Gln Met Glu Leu Leu His
    130                 135                 140

Ser Asn Gly Ser Gln Arg Thr Cys Cys Pro Val Asn Trp Val Glu His
145                 150                 155                 160

Gln Gly Ser Cys Tyr Trp Phe Ser His Ser Gly Lys Ala Trp Ala Glu
                165                 170                 175

Ala Glu Lys Tyr Cys Gln Leu Glu Asn Ala His Leu Val Val Ile Asn
            180                 185                 190

Ser Trp Glu Glu Gln Lys Phe Ile Val Gln His Thr Asn Pro Phe Asn
        195                 200                 205

Thr Trp Ile Gly Leu Thr Asp Ser Asp Gly Ser Trp Lys Trp Val Asp
    210                 215                 220

Gly Thr Asp Tyr Arg His Asn Tyr Lys Asn Trp Ala Val Thr Gln Pro
225                 230                 235                 240

Asp Asn Trp His Gly His Glu Leu Gly Gly Ser Glu Asp Cys Val Glu
                245                 250                 255

Val Gln Pro Asp Gly Arg Trp Asn Asp Asp Phe Cys Leu Gln Val Tyr
            260                 265                 270

Arg Trp Val Cys Glu Lys Arg Arg Asn Ala Thr Gly Glu Val Ala
        275                 280                 285

<210>  6

<211>  292
<212>  PRT
<213>  homo sapiens

<400>  6

Met Ala Lys Asp Phe Gln Asp Ile Gln Gln Leu Ser Ser Glu Glu Asn
1               5                   10                  15

Asp His Pro Phe His Gln Gly Pro Pro Pro Ala Gln Pro Leu Ala Gln
            20                  25                  30

Arg Leu Cys Ser Met Val Cys Phe Ser Leu Leu Ala Leu Ser Phe Asn
        35                  40                  45

Ile Leu Leu Leu Val Val Ile Cys Val Thr Gly Ser Gln Ser Glu Gly
    50                  55                  60

His Arg Gly Ala Gln Leu Gln Ala Glu Leu Arg Ser Leu Lys Glu Ala
65                  70                  75                  80

Phe Ser Asn Phe Ser Ser Ser Thr Leu Thr Glu Val Gln Ala Ile Ser
                85                  90                  95

Thr His Gly Gly Ser Val Gly Asp Lys Ile Thr Ser Leu Gly Ala Lys
            100                 105                 110

Leu Glu Lys Gln Gln Gln Asp Leu Lys Ala Asp His Asp Ala Leu Leu
        115                 120                 125

Phe His Leu Lys His Phe Pro Val Asp Leu Arg Phe Val Ala Cys Gln
    130                 135                 140

Met Glu Leu Leu His Ser Asn Gly Ser Gln Arg Thr Cys Cys Pro Val
145                 150                 155                 160

Asn Trp Val Glu His Gln Gly Ser Cys Tyr Trp Phe Ser His Ser Gly
                165                 170                 175

Lys Ala Trp Ala Glu Ala Glu Lys Tyr Cys Gln Leu Glu Asn Ala His
            180                 185                 190

Leu Val Val Ile Asn Ser Trp Glu Glu Gln Lys Phe Ile Val Gln His
        195                 200                 205

Thr Asn Pro Phe Asn Thr Trp Ile Gly Leu Thr Asp Ser Asp Gly Ser
    210                 215                 220

Trp Lys Trp Val Asp Gly Thr Asp Tyr Arg His Asn Tyr Lys Asn Trp
225                 230                 235                 240

Ala Val Thr Gln Pro Asp Asn Trp His Gly His Glu Leu Gly Gly Ser
                245                 250                 255

Glu Asp Cys Val Glu Val Gln Pro Asp Gly Arg Trp Asn Asp Asp Phe
            260                 265                 270

Cys Leu Gln Val Tyr Arg Trp Val Cys Glu Lys Arg Arg Asn Ala Thr
        275                 280                 285

Gly Glu Val Ala
    290

<210>  7
<211>  864
<212>  DNA
<213>  homo sapiens

<400>  7
atggccaagg actttcaaga tatccagcag ctgagctcgg aggaaaatga ccatcctttc      60

catcaagggc cacctcctgc ccagcccctg gcacagcgtc tctgctccat ggtctgcttc     120

agtctgcttg ccctgagctt caacatcctg ctgctggtgg tcatctgtgt gactgggtcc     180

caaagtgcac agctgcaagc cgagctgcgg agcctgaagg aagctttcag caacttctcc     240

tcgagcaccc tgacggaggt ccaggcaatc agcacccacg gaggcagcgt gggtgacaag     300

atcacatccc taggagccaa gctggagaaa cagcagcagg acctgaaagc agatcacgat     360

gccctgctct tccatctgaa gcacttcccc gtggacctgc gcttcgtggc ctgccagatg     420

gagctcctcc acagcaacgg ctcccaaagg acctgctgcc ccgtcaactg ggtggagcac     480

caaggcagct gctactggtt ctctcactcc gggaaggcct gggctgaggc ggagaagtac     540

tgccagctgg agaacgcaca cctggtggtc atcaactcct gggaggagca gaaattcatt     600

gtacaacaca cgaacccctt caatacctgg ataggtctca cggacagtga tggctcttgg     660

aaatgggtgg atggcacaga ctataggcac aactacaaga actgggctgt cactcagcca     720

gataattggc acgggcacga gctgggtgga agtgaagact gtgttgaagt ccagccggat     780

ggccgctgga acgatgactt ctgcctgcag gtgtaccgct gggtgtgtga gaaaaggcgg     840

aatgccaccg gcgaggtggc ctga                                            864

<210>  8
<211>  879
<212>  DNA
<213>  homo sapiens

<400>  8
atggccaagg actttcaaga tatccagcag ctgagctcgg aggaaaatga ccatcctttc      60

catcaagggc cacctcctgc ccagcccctg gcacagcgtc tctgctccat ggtctgcttc     120

agtctgcttg ccctgagctt caacatcctg ctgctggtgg tcatctgtgt gactgggtcc     180

caaagtgagg gtcacagagg tgcacagctg caagccgagc tgcggagcct gaaggaagct     240

ttcagcaact tctcctcgag caccctgacg gaggtccagg caatcagcac ccacggaggc     300

agcgtgggtg acaagatcac atccctagga gccaagctgg agaaacagca gcaggacctg     360

aaagcagatc acgatgccct gctcttccat ctgaagcact tccccgtgga cctgcgcttc 420
gtggcctgcc agatggagct cctccacagc aacggctccc aaaggacctg ctgccccgtc 480
aactgggtgg agcaccaagg cagctgctac tggttctctc actccgggaa ggcctgggct 540
gaggcggaga agtactgcca gctggagaac gcacacctgg tggtcatcaa ctcctgggag 600
gagcagaaat tcattgtaca acacacgaac cccttcaata cctggatagg tctcacggac 660
agtgatggct cttggaaatg ggtggatggc acagactata ggcacaacta caagaactgg 720
gctgtcactc agccagataa ttggcacggg cacgagctgg gtggaagtga agactgtgtt 780
gaagtccagc cggatggccg ctggaacgat gacttctgcc tgcaggtgta ccgctgggtg 840
tgtgagaaaa ggcggaatgc caccggcgag gtggcctga 879